# EUROPEAN PATENT SPECIFICATION

(11) **EP 4 099 986 B1**
(45) Date of publication and mention of the grant of the patent: **04.06.2025**
(21) Application number: 21711634.2
(22) Date of filing: 05.02.2021
(51) Int. Cl.: A61K 9/06, A61K 47/36, A61P 27/02, A61K 45/06, A61K 31/573, A61K 31/7048

(54) **XANTHAN-BASED OPHTHALMIC TOPICAL FORMULATIONS WITH A REDUCED DOSAGE REGIMEN**
OPHTHALMISCHE TOPISCHE FORMULIERUNGEN AUF XANTHANBASIS MIT REDUZIERTEM DOSIERUNGSSCHEMA
FORMULATIONS TOPIQUES OPHTALMIQUES À BASE DE XANTHANE AYANT UN RÉGIME POSOLOGIQUE RÉDUIT

(30) Priority: 06.02.2020 IT 202000002296
(43) Date of publication of application: 14.12.2022
(73) Proprietor: SIFI S.p.A., 95025 Aci Sant'Antonio (CT) (IT)
(72) Inventor: MAZZONE, Maria Grazia, 95025 Aci Sant'Antonio (IT); CIVIALE, Claudine, 95022 Acicatena (IT); SUDANO ROCCARO, Andrea, 95024 Acireale (IT); SOLFATO, Elena, 95030 Mascalucia (IT); ABBATE, Ilenia, 95024 Acireale (IT); CURATOLO, Maria Cristina, 95029 Viagrande (IT)
(74) Representative: Cattaneo, Elisabetta
(86) International application number: PCT/IB2021/050951
(87) International publication number: WO 2021/156813

(56) References cited:
- EP-A1- 1 913 948
- EP-B1- 1 913 948
- WO-A1-2010/004594
- WO-A1-2011/162752
- WO-A2-2019/166926
- IN-A- 5270 CH2 013
- US-A1- 2017 049 735
- US-B1- 6 264 935
- FARALDI FRANCESCO ET AL: "A new eye gel containing sodium hyaluronate and xanthan gum for the management of post-traumatic corneal abrasions", CLINICAL OPHTHALMOLOGY (AUCKLAND, N.Z.),, vol. 6, 9 May 2012 (2012-05-09), pages 727 - 731, XP009187022, ISSN: 1177-5483, DOI: 10.2147/OPTH.S31776
- ANONYMOUS: "Clinical Study to Evaluate the Efficacy and Tolerability of an Anti-inflammatory/Antibiotic Treatment Following Ocular Cataract Extraction - Tabular View - ClinicalTrials.gov", 25 November 2016 (2016-11-25), XP055802519, Retrieved from the Internet <URL:https://clinicaltrials.gov/ct2/show/record/NCT02973880?view=record> [retrieved on 20210507]

## Description

### FIELD OF THE INVENTION

The present invention applies to the medical field and, in particular, it relates to formulations for ophthalmic use characterised by a reduced dosage regimen.

### STATE OF THE ART

The treatment of eye pathologies by administering ophthalmic topical formulations may be a problem due to the limited contact time of the formulations, which are washed away rapidly due to eye and eyelid movements.

Such problem may be only partially overcome by increasing the administration frequency of the formulation, which can be a discomfort for the patient, resulting in a compliance reduction.

Research has been ongoing for formulations adapted for ophthalmic topical administration that allow a prolonged contact time and a greater efficacy of the active ingredient contained therein.

### SUMMARY OF THE INVENTION

The inventors of the present Patent Application have surprisingly found out that a base ophthalmic formulation comprising xanthan gum allows reducing the dosage regimen and increasing synergically the efficacy of the active ingredient.

The present invention, in one first object thereof, relates to ophthalmic topical formulations comprising xanthan gum for medical use in the treatment of eye pathologies, characterised in that they are administered to a patient according to a reduced dosage regimen as claimed in Claim 1.

In particular, the present invention, in one aspect thereof, relates to an ophthalmic topical formulation comprising xanthan gum in an amount ranging from 0.7 to 5.0% (weight/volume) and netilmicin or salts thereof, for use in the treatment of an eye pathology having an inflammatory condition of the front segment of the eye in the presence of a bacterial infection, wherein said formulation is administered to a patient in need thereof twice a day or once a day.

The inventors of the present Application have in fact surprisingly found out that xanthan gum, if used in an ophthalmic formulation shows surprisingly a series of properties such to allow reducing the dosage regimen and increasing synergically the efficacy of the active ingredient. In particular, the xanthan gum acts as a mucomimetic substance, as it prevents the dehydration of the eye surface and maintains the integrity of the corneal epithelium. It reduces friction between eyelid movements. By interacting with the tear and the eye surface it contributes to the cellular homeostasis, and promotes eye adsorption, enhancing the efficacy of the active ingredient and increasing the eye tolerability of the formulation.

Thanks to these mucomimetic and eye adsorption promoting properties, the formulation according to the present invention can be thus administered to a patient in need thereof with a dosage regimen that is reduced if compared to a similar formulation of the background art, maintaining at the same time the same levels of therapeutic efficacy. Reducing the discomfort for the patient, this results in an increase in the therapy compliance.

According to a particular aspect of the invention, the xanthan gum is comprised in a base formulation.

The xanthan gum-based formulations comprise one or more further active ingredients. According to the invention, the formulations comprise netilmicin or salts thereof.

According to a preferred aspect, further active ingredients are represented by anti-inflammatory, antibiotic, anti-glaucoma and anti-allergic active ingredients.

In a further aspect, the present invention relates to an ophthalmic topical formulation comprising xanthan gum, netilmicin and dexamethasone for use in the treatment of an eye pathology having an inflammatory condition of the front segment of the eye in the presence of a bacterial infection, wherein said formulation comprises xanthan gum in an amount ranging from 0.7 to 5.0% (weight/volume) and wherein said formulation is administered to a patient in need thereof with a dose of 0.100 to 0.540 mg/day of netilmicin and of 0.03 to 0.18 mg/day of dexamethasone.

Thanks to the mucomimetic and eye adsorption promoting properties of the xanthan gum noted with reference to the first aspect of the invention, in fact, it is possible to administer to a patient in need thereof an ophthalmic formulation comprising netilmicin and dexamethasone, with a reduced dosage regimen.

The present description, in further aspects thereof, refers to the use of xanthan gum as a mucomimetic substance and as an eye adsorption promoter, in the treatment of an eye pathology.

The advantages and the characteristics of said further aspects have already been underlined with reference to the first aspect of the invention and will not be herein repeated.

### BRIEF DESCRIPTION OF THE DRAWINGS

Figure 1 shows the results of the in vitro toxicity essays for A) BME -negative control; B) SDS -positive control; C) Carbopol 980; D) Lutrol F127; E) Xanthan gum;
Figure 2 shows the results of cell viability essay on SIRC cells of the xanthan gum;
Figure 3 shows the results of QI determined for netilmicin eye drop in single dose against conjunctival eye strains (A) MSSA, (B) MRSA, (C) MSCoNS, (D) MRCoNS;
Figure 4 shows the results of QI determined for netilmicin gel in single dose against conjunctival eye strains (A) MSSA, (B) MRSA, (C) MSCoNS, (D) MRCoNS;
Figure 5 shows the results of QI determined for netilmicin eye drop in single dose against corneal eye strains (A) MSSA, (B) MRSA, (C) MSCoNS, (D) MRCoNS; and
Figure 6 shows the results of QI determined for netilmicin gel in single dose against corneal eye strains (A) MSSA, (B) MRSA, (C) MSCoNS, (D) MRCoNS.

### DETAILED DESCRIPTION OF THE INVENTION

The present invention, in one first object thereof, relates to ophthalmic topical formulations comprising xanthan gum for medical use in the treatment of eye pathologies, characterised in that they are administered to a patient according to a reduced dosage regimen as claimed in claim 1.

In particular, the present invention, in one aspect thereof, relates to an ophthalmic topical formulation comprising xanthan gum in an amount ranging from 0.7 to 5.0% (weight/volume) and netilmycin or salts thereof for use in the treatment of an eye pathology having an inflammatory condition of the front segment of the eye in the presence of a bacterial infection, wherein said formulation is administered to a patient in need thereof twice a day or once a day.

The inventors of the present Application have in fact surprisingly found out that xanthan gum, if used in an ophthalmic formulation shows surprisingly a series of properties such to allow reducing the dosage regimen and increasing synergically the efficacy of the active ingredient. In particular, the xanthan gum shows in fact mucomimetic properties, interacting with the tear and the eye surface so as to contribute to cellular homeostasis, and eye adsorption promoting properties, enhancing the efficacy of the active ingredient and improving the eye tolerability of the formulation itself.

Thanks to these mucomimetic and eye adsorption promoting properties, the formulation according to the present invention can be thus administered to a patient in need thereof with a dosage regimen that is reduced if compared to a similar formulation of the background art, maintaining at the same time the same levels of therapeutic efficacy. Reducing the discomfort for the patient, this results in an increase in the therapy compliance.

Within the context of the present description and following claims, all the numerical magnitudes indicating quantities, parameters, percentages, and so on are to be considered preceded in every circumstance by the term "about" unless indicated otherwise. Further, all the ranges of numerical magnitudes include all the possible combinations of maximum and minimum numerical values and all the possible intermediate ranges, as well as those indicated below.

The present invention can be presented in one or more of its aspects or one or more of the preferred characteristics reported below, which can be combined with one another as preferred according to the application requirements.

For the purposes of the present invention, within the disclosed formulations the xanthan gum (xanthan) has a mucomimetic effect and interacts with the tear and eye surface, thus contributing to cellular homeostasis, and has an eye adsorption promoting effect, enhancing the efficacy of the active ingredient and improving the eye tolerability of the formulation itself.

Preferably, said xanthan gum is used in the formulation according to the present invention as a mucomimetic substance.

Preferably, said xanthan gum is used in the formulation according to the present invention as an eye adsorption promoter.

The xanthan gum within the formulation according to the present invention may be any xanthan gum commercially known and suitable for the use in ophthalmic applications. Examples of xanthan gum which can be used in the present invention are Xanthan gum NF grade, Xantural^{®} 180, Xanthan gum NF-C grade Vanzan, Xantural^{®} 75 products.

Preferably, the formulation according to the present invention comprises 0.8 to 3.5 % (weight/volume), more preferably 0.85 to 2 % (weight/volume) of xanthan gum. According to a particular aspect of the invention, the xanthan gum is comprised in a base formulation.

The ophthalmic topical formulation according to the invention comprises netilmicin as additional active ingredient. Preferably, the ophthalmic topical formulation may further comprise one or more active ingredients selected from the group consisting of: steroidal anti-inflammatory drugs, non-steroidal anti-inflammatory drugs, antiallergic drugs, beta blockers, prostaglandin inhibitors, carbonic anhydrase inhibitor, alpha-adrenergic agonists, antiviral drugs, antibiotics, anaesthetics and mydriatics. Preferably, said active ingredients have a log P ≤ 2.5.

In one preferred embodiment, the ophthalmic topical formulation according to the present invention further comprises one or more antibiotics. More preferably, such antibiotic is selected from the group consisting in: levofloxacin, ofloxacin, tobramycin, azithromycin and the derivative salts thereof. According to the invention the formulation comprises netilmicin or a derivative salt thereof.

A preferred example of derivative salt of netilmicin is netilmicin sulfate.

Preferably, the ophthalmic topical formulation according to the present invention is administered to a patient in need thereof with a dose of 0.100 - 0.540 mg/day, more preferably of 0.160 to 0.320 mg/day, still more preferably of 0.210 to 0.270 mg/day, of netilmicin.

The ophthalmic topical formulation according to the present invention comprises 0.1 to 0.5% (weight/volume), and more preferably 0.2 to 0.4% (weight/volume) of netilmicin. In an aspect of the present invention, the disclosed formulations comprise a non-steroidal anti-inflammatory drug. More preferably, such anti-inflammatory drug is represented by dexamethasone, bromfenac, nepafenac, derivative salts thereof and, in another still more preferred aspect, is represented by dexamethasone or a derivative salt thereof.

Preferred examples of derivative salts of dexamethasone are: dexamethasone disodium phosphate, dexamethasone acetate, dexamethasone isonicotinate, dexamethasone metasulfobenzoate.

Preferably, the ophthalmic topical formulation according to the present invention is administered to a patient in need thereof with a dose 0.03 - 0.18 mg/day, more preferably 0.05 to 0.11 mg/day, still more preferably 0.07 to 0.09 mg/day, of dexamethasone.

Preferably, the ophthalmic topical formulation according to the present invention comprises 0.02 to 0.4 % (weight/volume), and still more preferably 0.05 to 0.2 % (weight/volume) of dexamethasone.

Thanks to the properties of the xanthan gum according to the present invention, the ophthalmic topical formulation according to the present invention may be advantageously used for treating an pathology showing an inflammatory state of the front segment of the eye, either after surgery or not, in the presence or with a risk of bacterial infection, preferably for treating an eye pathology selected from the group consisting of: an eye infection, such as preferably blepharitis, bacterial conjunctivitis, and keratitis, a trauma or after-surgery eye abrasion, and eye dryness.

Preferably, the ophthalmic topical formulation for use according to the present invention is administered to a patient in need thereof for a period of at least 7 days, more preferably for a period ranging from 7 to 21 days.

In a particularly preferred embodiment, the ophthalmic topical formulation according to the present invention comprises netilmicin and dexamethasone, or derivative salts thereof.

The ophthalmic topical formulation according to said particularly preferred embodiment has one or more of the above-mentioned characteristics with reference to said active principles.

Thanks to the properties of the xanthan gum according to the present invention, the ophthalmic topical formulation comprising netilmicin and dexamethasone may be advantageously used for treating inflammatory states of the front segment of the eye, either after surgery or not, in the presence or with a risk of bacterial infection, preferably for treating an eye pathology selected from the group consisting of: eye infections, such as preferably blepharitis, bacterial conjunctivitis, and keratitis, trauma or after-surgery eye abrasions.

In the case of the ophthalmic formulation comprising netilmicin and dexamethasone, this is preferably administered to a patient in need thereof for a period of at least 7 days, more preferably for a period ranging from 7 to 15 days.

The ophthalmic topical formulation according to the present invention may contain one or more further components known in the field of ophthalmic formulations.

Preferably, the ophthalmic topical formulation according to the present invention comprises at least a further component selected from the group consisting of: a buffer, an isotonizing agent, hyaluronic acid or a pharmaceutically acceptable salt thereof.

Preferably, said buffer is selected from the group consisting of: phosphate buffer, citrate buffer, phosphate/citrate buffer, tris buffer, or any phosphate-free buffer.

Preferably, said isotonizing agent is selected from the group consisting of ionic salts and non-ionic molecules.

Preferably, said ionic salts are selected from the group consisting of: sodium citrate, sodium chloride, potassium chloride, magnesium chloride and calcium chloride.

Preferably, said non-ionic molecules are selected from the group consisting of; glycerol, mannitol and polyethylene glycol.

The formulation according to the present invention is preferably aqueous-based and still more preferably in gel form.

In a further aspect, the present invention relates to an ophthalmic topical formulation comprising xanthan gum, netilmicin and dexamethasone for use in the treatment of an eye pathology having an inflammatory condition of the front segment of the eye in the presence of a bacterial infection, wherein said formulation comprises xanthan gum in an amount ranging from 0.7 to 5.0% (weight/volume) and wherein said formulation is administered to a patient in need thereof with a dose of 0.100 to 0.540 mg/day of netilmicin and of 0.03 to 0.18 mg/day of dexamethasone.

Thanks to the mucomimetic and eye adsorption promoting properties of the xanthan gum noted with reference to the first aspect of the invention, in fact, it is possible to administer to a patient in need thereof an ophthalmic formulation comprising netilmicin and dexamethasone, with a reduced dosage regimen.

The ophthalmic topical formulation according to a further aspect of the invention preferably has one or more of the above-mentioned characteristics with reference to the formulation according to the first aspect of the invention.

The ophthalmic topical formulation according to this further aspect of the invention is administered to a patient in need thereof with a dosage regimen of preferably maximum twice a day, and may be thus advantageously administered also once a day.

Preferably, said formulation is administered to said patient in need thereof with a dose of 0.160 to 0.320 mg/day, more preferably of 0.210 to 0.270 mg/day, of netilmicin.

Preferably, the ophthalmic topical formulation according to this further aspect of the invention comprises 0.1 to 0.5% (weight/volume), and more preferably 0.2 to 0.4. % (weight/volume) of netilmicin.

Preferably, the ophthalmic topical formulation according to this further aspect of the invention is administered to a patient in need thereof with a dose of 0.05 to 0.11 mg/day, more preferably of 0.07 to 0.09 mg/day, of dexamethasone.

Preferably, the ophthalmic topical formulation according to this further aspect of the invention comprises 0.02 to 0.4 % (weight/volume), and still more preferably 0.05 to 0.2 % (weight/volume) of dexamethasone.

Preferably, the ophthalmic topical formulation according to this further aspect of the invention may be advantageously used for treating an eye pathology having an inflammatory state of the front segment of the eye, either after surgery or not, in the presence or with a risk of bacterial infection, more preferably for treating an eye illness selected from the group consisting of: an eye infection, such as preferably blepharitis, bacterial conjunctivitis, and keratitis, a trauma or after-surgery eye abrasion.

Preferably, the ophthalmic topical formulation for use according to this further aspect of the invention is administered to a patient in need thereof for a period of at least 7 days, more preferably for a period ranging from 7 to 15 days.

According to what above described, for exemplary purposes, the following ophthalmic topical formulations are reported:

### Formulation 1 (not according to the invention)

| ***Component*** | ***Amount % (weight*/*volume)*** |
|---|---|
| Xanthan gum | 1.0000 |
| Sodium hyaluronate | 0.1500 |
| Sodium hydrogen phosphate monohydrate | 0.1600 |
| Disodium phosphate dodecahydrate | 0.4100 |
| Sodium citrate | 0.0590 |
| Sodium chloride | 0.3500 |
| Potassium chloride | 0.1500 |
| Magnesium chloride hexahydrate | 0.0120 |
| Calcium chloride dihydrate | 0.0084 |
| Glycerol | 0.5000 |
| Purified water | q.s. to 100 ml |

### Formulation 2

| ***Component*** | ***Amount % (weight*/*volume)*** |
|---|---|
| Netilmicin and salts thereof | 0.30 as base |
| Dexamethasone and salts thereof | 0.10 as alcohol |
| Xanthan gum | 0.7-5.0 |
| Sodium hydrogen phosphate monohydrate | 0.1465 |
| Disodium phosphate dodecahydrate | 0.5000 |
| Sodium citrate | 2.1000 |
| Purified water | q.s. to 100 ml |

### Formulation 3 (not according to the invention)

| ***Component*** | ***Amount % (weight*/*volume)*** |
|---|---|
| Dexamethasone and salts thereof | 0.10 as alcohol |
| Xanthan gum | 0.7-5.0 |
| Buffer | q.s. to 100 ml |
| Isotonizing agents | |
| Purified water | |

### Formulation 4

| ***Component*** | ***Amount % (*weight/volume*)*** |
|---|---|
| Netilmicin and salts thereof | 0.3 as base |
| Xanthan gum | 0.7-5.0 |
| Viscosity-inducing agents | q.s. to 100 ml |
| Buffer | |
| Isotonizing agents | |
| Purified water | |

The present description, in further aspects thereof, refers to the use of xanthan gum as a mucomimetic substance and as an eye adsorption promoter, in the treatment of an eye pathology.

The advantages and the characteristics of said further aspects have already been underlined with reference to the first aspect of the invention and will not be herein repeated.

The invention is now shown by means of some Examples to be considered for exemplary and non-limiting purposes thereof.

### EXPERIMENTAL PART

### Example 1

In order to demonstrate the efficacy in the reduction of the dosage regimen and the safety of the xanthan gum-based ophthalmic formulation a clinical trial was made for the formulation containing netilmicin/dexamethasone in a xanthan gum polymer matrix. In the detail of the invention the reduction of the dosage regimen provides administering netilmicin/dexamethasone in the xanthan gum-based formulation twice a day (BID, bis in die, with a dose of 0.240 mg/day of netilmicin and 0.08 mg/day of dexamethasone) if compared to the eye drop four times a day (QID, quarter in die, with a dose of 0.480 mg/day of netilmicin and 0.16 mg/day of dexamethasone), for the treatment of after-surgery cataract inflammatory conditions, in the presence or with a risk of microbial infection.

The following formulations, in particular, were tested:

### Xanthan gum-based gel formulation:

| ***Component*** | ***Amount % (weight*/*volume)*** |
|---|---|
| Netilmicin sulfate | 0.30 as base |
| Dexamethasone disodium phosphate | 0.10 as alcohol |
| Xanthan gum | 1 |
| Sodium hydrogen phosphate monohydrate | 0.1465 |
| Disodium phosphate dodecahydrate | 0.5000 |
| Sodium citrate | 2.1000 |
| Purified water | q.s. to 100 ml |

### Reference Formulations for eye drops:

| ***Component*** | ***Amount % (weight*/*volume)*** |
|---|---|
| Netilmicin sulfate | 0.30 as base |
| Dexamethasone disodium phosphate | 0.10 as alcohol |
| Sodium hydrogen phosphate monohydrate | 0.1465 |

| | |
|---|---|
| Disodium phosphate dodecahydrate | 0.5000 |
| Sodium citrate | 2.1000 |
| Purified water | q.s. to 100 ml |

The trial, defined as a step III International multi-centre (n=5), double blind and non-inferiority trial, has provided recruiting 168 patients, male and female, aged over 40, who were randomised to receive BID the ophthalmic netilmicin/dexamethasone in a xanthan gum-based gel formulation (N = 85) or QID the eye drop formulation (N = 83) from the day of the cataract surgery (Day 0), until Day 15.

The percentage of complete responders (patients without cells and flare in the front chamber) after 7 days was assessed as a primary endpoint. Secondary parameters (microbial measuring, IOP, evaluation of clinical signs/symptoms, VA and safety) were assessed at each examination.

The results obtained demonstrated that flare signs and lack of cells (cellularity) were solved at day 7 after surgery in 92.5% of patients and completely within day 15. Both in ITT (Intent to Treat) and in PP (per protocol) populations, the efficacy analysis demonstrated that the netilmicin/dexamethasone association administered twice a day in a xanthan gum-based gel formulation is not lower than that in an eye drop formulation administered four times a day.

Evidence has demonstrated that in the clinical response obtained after 7 days, the xanthan gum-based gel formulation allowed to reduce the dosage regimen (twice a day) surprisingly demonstrating non-inferiority if compared to the dosage regimen approved for eye drops (4 times a day). Both treatments were very well tolerated and effective.

To the benefit of the invention, the xanthan gum-based formulation was evaluated comparing it with other compositions having a different polymer nature.

In particular the in vitro safety profile and in vivo tolerability were assessed for xanthan gum-based formulations, carbopol 980 and lutrol F127.

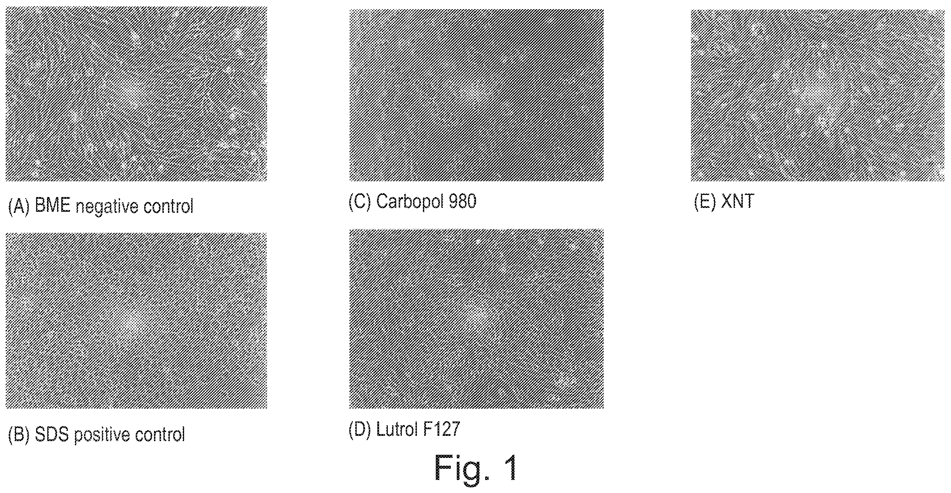

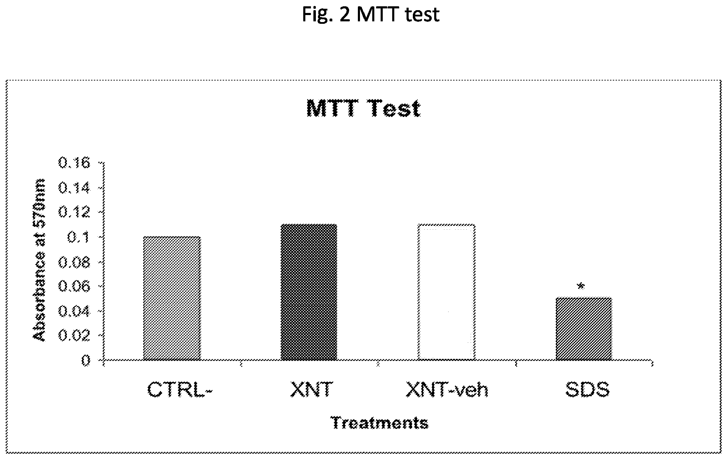

In the results of the acute (8 administrations per day) and sub-acute (5 administrations per day) Draize test, the formulation in a xanthan gum polymer matrix demonstrated an excellent performance with no cell toxic effects, as demonstrated by the cell morphology and by the viability test MTT (Figures 1 and 2).

To support the invention, an analysis for determining the QIs of levofloxacin, ofloxacin, netilmicin, tobramycin and azithromycin was also carried out. In detail the maximum effective concentration (Cmax) and the minimum inhibitory concentration (MIC90) were assessed on the bacterial strains most widespread in the corneal and conjunctival eye infections (MSSA, MRSA, MSCoNS, MRCoNS).

The obtained MIC90 and Cmax data were related to each other to calculate QI in both tissues.

In particular, Cmax corneal and conjuctival values for netilmicin, tobramycin, levofloxacin, ofloxacin and azithromycin, in single or multiple dose, were extracted from several in vivo pharmacokinetics studies.

MIC90 values for the five antibiotics being examined were obtained from two studies which completely match the selection criteria.

Cmax values were related to MIC 90 values and the QIs obtained were plotted in a chart.

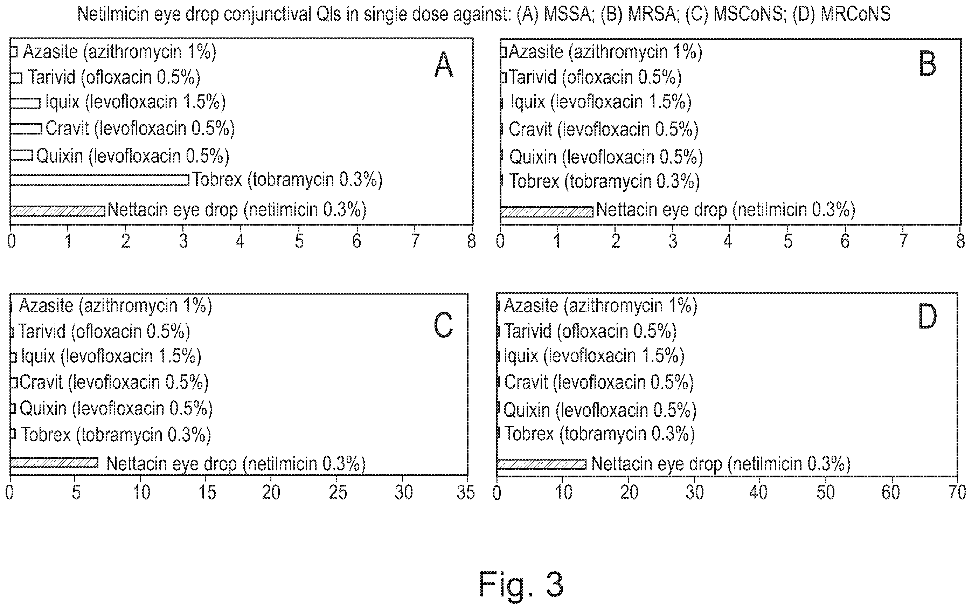

QI values show that, as regards the single dose, the netilmicin-containing eye drop revealed to be the best in conjunctiva against bacterial strains MRSA, MRCoNS and MSCoNS (Fig.3).

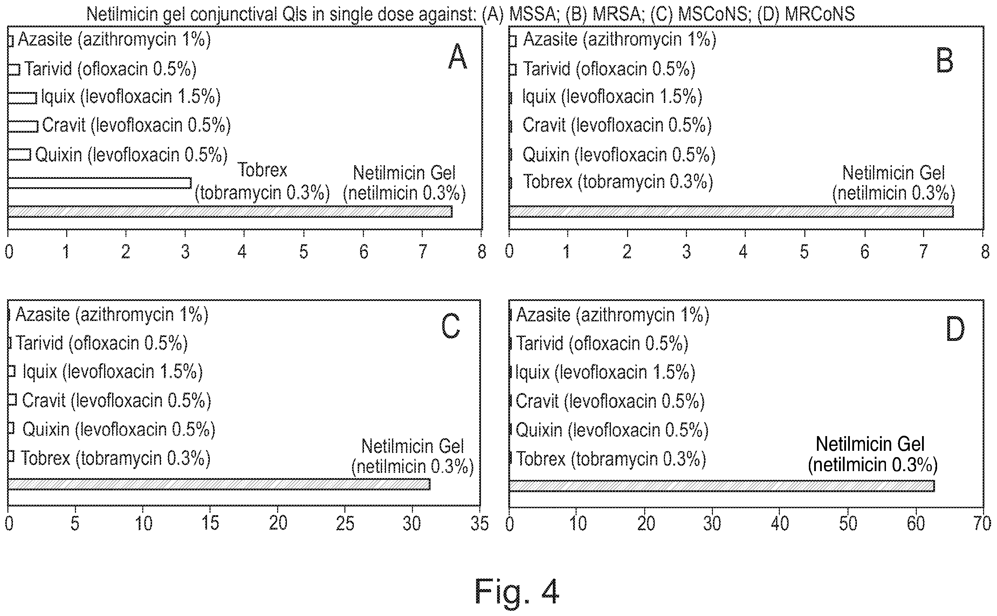

It is important to note that, under the same conditions, the xanthan-based netilmicin gel formulation allowed a significant general increase of QIs, considering that, even against MSSA, it revealed to be the most effective formulation among those studied (Fig.4).

In the cornea, the netilmicin eye drop showed higher QIs than other formulations in MRSA and MRCoNS strains.

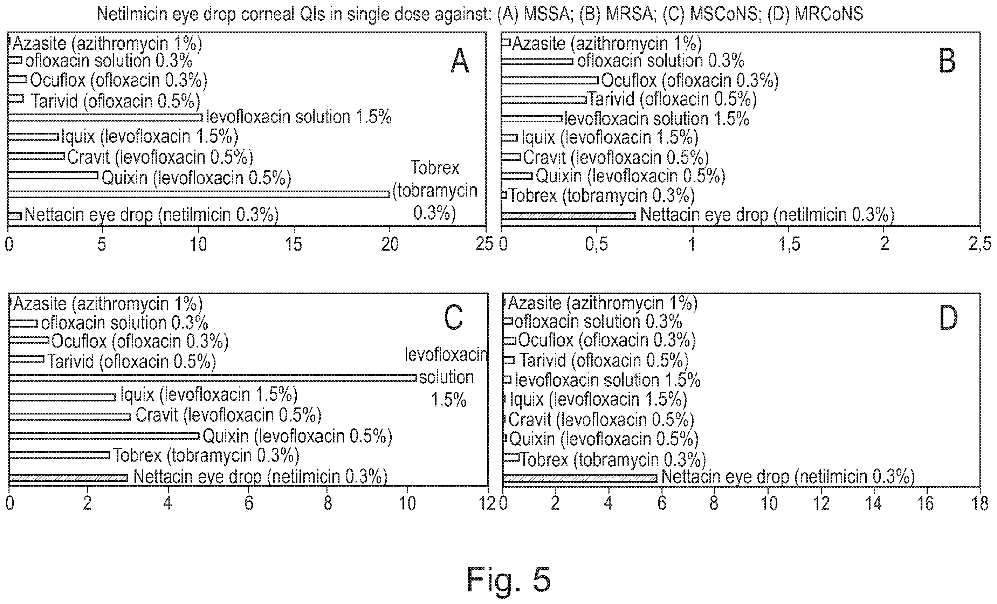

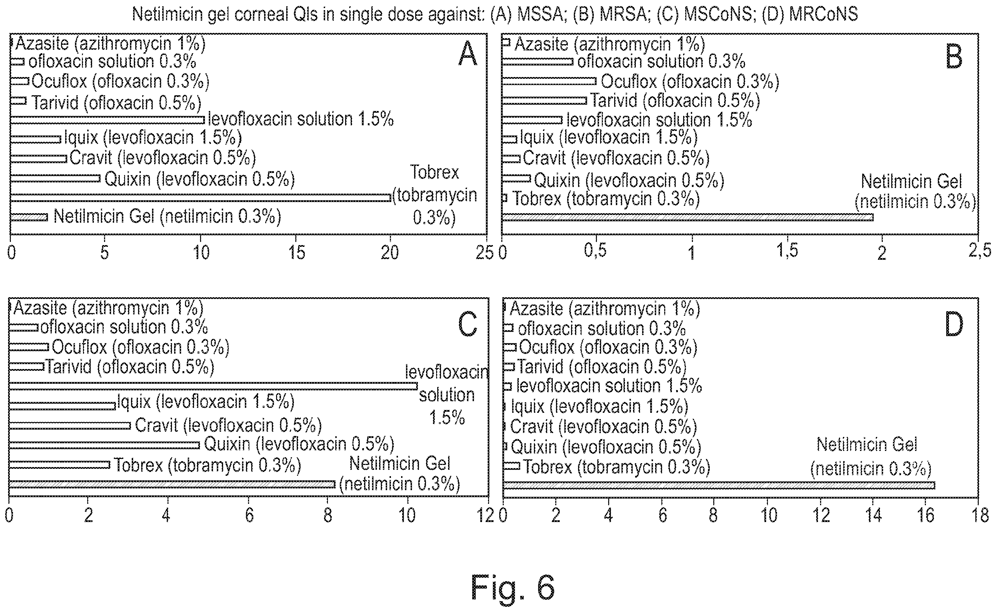

It also performed better than formulations containing azithromycin and some quinolones in MSCoNS (Fig.5). Equally surprising in this case, under the same conditions, netilmicin gel formulation determined a significant increase in QIs so that, apart from a 1.5% lovofloxacin solution (not used in ophthalmic traded products), netalmicin gel revealed to be the most effective formulation against MSCoNS. Against MSSA, netilmicin gel reached better corneal QI values than azithromycin (Azasite^{®}) and ofloxacin formulations (Fig.6).

Such evidence supports the invention, demonstrating that in the netilmicin gel formulation, the molecule is able to interact with the xanthan-based polymer matrix significantly enhancing its strength and promoting the increase of QIs inside target tissues.

## Claims

1. An ophthalmic topical formulation comprising xanthan gum in an amount ranging from 0.7 to 5.0% (weight/volume) and netilmicin or salts thereof, for the use in the treatment of an eye pathology having an inflammatory condition of the front segment of the eye in the presence of a bacterial infection, wherein said formulation is administered to a patient in need thereof twice a day or once a day.

2. The ophthalmic topical formulation for use according to claim 1, wherein said formulation further comprises one or more active ingredients selected from the group consisting of: steroidal anti-inflammatory drugs, non-steroidal anti-inflammatory drugs, antiallergic drugs, beta blockers, prostaglandin inhibitors, carbonic anhydrase inhibitor, alpha-adrenergic agonists, antiviral drugs, antibiotics selected from the group consisting of: levofloxacin, ofloxacin, tobramycin, azithromycin and salts thereof, anaesthetics and mydriatics.

3. The ophthalmic topical formulation for use according to claim 1, wherein said formulation is administered to said patient with a dose of 0.100 to 0.540 mg/day of netilmicin.

4. The ophthalmic topical formulation for use according to any one of claims 1 to 3, comprising 0.1 to 0.5% (weight/volume) of netilmicin.

5. The ophthalmic topical formulation for use according to any one of claims 2 to 4, wherein said one or more anti-inflammatory drugs are selected from the group consisting of: dexamethasone, bromfenac, nepafenac, and salts thereof.

6. The ophthalmic topical formulation for use according to claim 5, wherein one or more steroidal anti-inflammatory drugs are dexamethasone and salts thereof.

7. The ophthalmic topical formulation for use according to claim 6, wherein said formulation is administered to said patient with a dose of 0.03 to 0.18 mg/day of dexamethasone.

8. The ophthalmic topical formulation for use according to claim 6 or 7, comprising 0.02 to 0.4 % (weight/volume) of dexamethasone.

9. The ophthalmic topical formulation for use according to claim 1, wherein said eye pathology is selected from the group consisting of: an eye infection, a trauma or after-surgery eye abrasion, and eye dryness.

10. The ophthalmic topical formulation for use according to claim 9, wherein said eye infection is selected from the group consisting of: blepharitis, bacterial conjunctivitis, and keratitis.

11. The ophthalmic topical formulation for use according to any one of claims 1 to 10, wherein said formulation comprises netilmicin and dexamethasone or salts thereof.

12. An ophthalmic topical formulation comprising xanthan gum, netilmicin and dexamethasone for use in the treatment of an eye pathology having an inflammatory condition of the front segment of the eye in the presence of a bacterial infection, wherein said formulation comprises xanthan gum in an amount ranging from 0.7 to 5.0% (weight/volume) and wherein said formulation is administered to a patient in need thereof with a dose of 0.100 to 0.540 mg/day of netilmicin and of 0.03 to 0.18 mg/day of dexamethasone.

13. The ophthalmic topical formulation for use according to claim 12, comprising 0.1 to 0.5 % (weight/volume) of netilmicin.

14. The ophthalmic topical formulation for use according to claim 12 or 13, comprising 0.02 to 0.4 % (weight/volume) of dexamethasone.

15. The ophthalmic topical formulation for use according to claim 12, wherein said eye pathology is selected from the group consisting of: an eye infection, and a trauma or after-surgery eye abrasion.

16. The ophthalmic topical formulation for use according to claim 15, wherein said eye infection is selected from the group consisting of: blepharitis, bacterial conjunctivitis, and keratitis.

17. The ophthalmic topical formulation for use according to any one of claims 1 to 10, comprising:
| ***Component*** | ***Amount % (weight*/*volume)*** |
|---|---|
| Netilmicin and salts thereof | 0.30 as base |
| Dexamethasone and salts thereof | 0.10 as alcohol |
| Xanthan gum | 0.7-5.0 |
| Sodium hydrogen phosphate monohydrate | 0.1465 |
| Disodium phosphate dodecahydrate | 0.5000 |
| Sodium citrate | 2.1000 |
| Purified water | q.s. to 100 ml |
or comprising:
| ***Component*** | ***Amount % (weight*/*volume)*** |
|---|---|
| Netilmicin and salts thereof | 0.3 as base |
| Xanthan gum | 0.7-5.0 |
| Viscosity-inducing agents | q.s. to 100 ml |
| Buffer | |
| Isotonizing agents | |
| Purified water | |

18. The ophthalmic topical formulation for use according to any one of claims 12 to 16, comprising:
| ***Component*** | ***Amount % (weight*/*volume)*** |
|---|---|
| Netilmicin and salts thereof | 0.30 as base |
| Dexamethasone and salts thereof | 0.10 as alcohol |
| | |
|---|---|
| Xanthan gum | 0.7-5.0 |
| Sodium hydrogen phosphate monohydrate | 0.1465 |
| Disodium phosphate dodecahydrate | 0.5000 |
| Sodium citrate | 2.1000 |
| Purified water | q.s. to 100 ml |

## Patentansprüche

1. Ophthalmische topische Formulierung, die Xanthangummi in einer Menge im Bereich von 0,7 bis 5,0% (Gewicht/Volumen) und Netilmicin oder Salze davon umfasst, zur Verwendung bei der Behandlung einer Augenpathologie mit einem entzündlichen Zustand des vorderen Segments des Auges in Gegenwart einer bakteriellen Infektion, wobei die Formulierung einem Patienten, der sie benötigt, zweimal täglich oder einmal täglich verabreicht wird.

2. Ophthalmische topische Formulierung zur Verwendung nach Anspruch 1, wobei die Formulierung ferner einen oder mehrere Wirkstoffe umfasst, ausgewählt aus der Gruppe, bestehend aus: steroidalen Antirheumatika, nichtsteroidalen Antirheumatika, antiallergischen Arzneimitteln, Betablockern, Prostaglandinhemmem, Kohlensäureanhydrase-Hemmern, alpha-adrenergen Agonisten, antiviralen Arzneimitteln, Antibiotika, ausgewählt aus der Gruppe, bestehend aus: Levofloxacin, Ofloxacin, Tobramycin, Azithromycin und Salzen davon, Anästhetika und Mydriatika.

3. Ophthalmische topische Formulierung zur Verwendung nach Anspruch 1, wobei die Formulierung dem Patienten mit einer Dosis von 0,100 bis 0,540 mg/Tag Netilmicin verabreicht wird.

4. Ophthalmische topische Formulierung zur Verwendung nach einem der Ansprüche 1 bis 3, die 0,1 bis 0,5% (Gewicht/Volumen) Netilmicin umfasst.

5. Ophthalmische topische Formulierung zur Verwendung nach einem der Ansprüche 2 bis 4, wobei das eine oder die mehreren Antirheumatika ausgewählt sind aus der Gruppe, bestehend aus: Dexamethason, Bromfenac, Nepafenac und Salzen davon.

6. Ophthalmische topische Formulierung zur Verwendung nach Anspruch 5, wobei ein oder mehrere steroidale Antirheumatika Dexamethason und Salze davon sind.

7. Ophthalmische topische Formulierung zur Verwendung nach Anspruch 6, wobei die Formulierung dem Patienten mit einer Dosis von 0,03 bis 0,18 mg/Tag Dexamethason verabreicht wird.

8. Ophthalmische topische Formulierung zur Verwendung nach Anspruch 6 oder 7, die 0,02 bis 0,4% (Gewicht/Volumen) Dexamethason umfasst.

9. Ophthalmische topische Formulierung zur Verwendung nach Anspruch 1, wobei die Augenpathologie ausgewählt ist aus der Gruppe, bestehend aus: einer Augeninfektion, einem Trauma oder einem Augenabrieb nach einer Operation und Augentrockenheit.

10. Ophthalmische topische Formulierung zur Verwendung nach Anspruch 9, wobei die Augeninfektion ausgewählt ist aus der Gruppe, bestehend aus: Blepharitis, bakterieller Konjunktivitis und Keratitis.

11. Ophthalmische topische Formulierung zur Verwendung nach einem der Ansprüche 1 bis 10, wobei die Formulierung Netilmicin und Dexamethason oder Salze davon umfasst.

12. Ophthalmische topische Formulierung, die Xanthangummi, Netilmicin und Dexamethason umfasst, zur Verwendung bei der Behandlung einer Augenpathologie mit einem entzündlichen Zustand des vorderen Segments des Auges in Gegenwart einer bakteriellen Infektion, wobei die Formulierung Xanthangummi in einer Menge im Bereich von 0,7 bis 5,0% (Gewicht/Volumen) umfasst und wobei die Formulierung einem Patienten, der sie benötigt, mit einer Dosis von 0,100 bis 0,540 mg/Tag Netilmicin und 0,03 bis 0,18 mg/Tag Dexamethason verabreicht wird.

13. Ophthalmische topische Formulierung zur Verwendung nach Anspruch 12, die 0,1 bis 0,5% (Gewicht/Volumen) Netilmicin umfasst.

14. Ophthalmische topische Formulierung zur Verwendung nach Anspruch 12 oder 13, die 0,02 bis 0,4% (Gewicht/Volumen) Dexamethason umfasst.

15. Ophthalmische topische Formulierung zur Verwendung nach Anspruch 12, wobei die Augenpathologie ausgewählt ist aus der Gruppe, bestehend aus: einer Augeninfektion und einem Trauma oder einem Augenabrieb nach einer Operation.

16. Ophthalmische topische Formulierung zur Verwendung nach Anspruch 15, wobei die Augeninfektion ausgewählt ist aus der Gruppe, bestehend aus: Blepharitis, bakterieller Konjunktivitis und Keratitis.

17. Ophthalmische topische Formulierung zur Verwendung nach einem der Ansprüche 1 bis 10, umfassend:
| Bestandteil | Menge % (GewichtNolumen) |
|---|---|
| Netilmicin und Salze davon | 0,30 als Base |
| Dexamethason und Salze davon | 0,10 als Alkohol |
| Xanthangummi | 0,7-5,0 |
| Natriumhydrogenphosphatmonohydra t | 0,1465 |
| Dinatriumphosphat-Dodecahydrat | 0,5000 |
| Natriumcitrat | 2,1000 |
| Gereinigtes Wasser | q.s. bis 100 ml |
oder umfassend:
| Bestandteil | Menge % (GewichtNolumen) |
|---|---|
| Netilmicin und Salze davon | 0,3 als Base |
| Xanthangummi | 0,7-5,0 |
| Viskositätsfördernde Mittel | q.s. bis 100 ml |
| Puffer | |
| Isotonisierungsmittel | |
| Gereinigtes Wasser | |

18. Ophthalmische topische Formulierung zur Verwendung nach einem der Ansprüche 12 bis 16, umfassend:
| Bestandteil | Menge % (GewichtNolumen) |
|---|---|
| Netilmicin und Salze davon | 0,30 als Base |
| Dexamethason und Salze davon | 0,10 als Alkohol |
| Xanthangummi | 0,7-5,0 |
| Natriumhydrogenphosphatmonohydra t | 0,1465 |
| Dinatriumphosphat-Dodecahydrat | 0,5000 |
| Natriumcitrat | 2,1000 |
| Gereinigtes Wasser | q.s. bis 100 ml |

## Revendications

1. Formulation topique ophtalmique comprenant de la gomme xanthane en une quantité allant de 0,7 à 5,0 % (poids/volume) et de la nétilmicine ou ses sels, pour l'utilisation dans le traitement d'une pathologie oculaire ayant un état inflammatoire du segment avant de l'œil en présence d'une infection bactérienne, dans laquelle ladite formulation est administrée à un patient qui en a besoin deux fois par jour ou une fois par jour.

2. Formulation topique ophtalmique à utiliser selon la revendication 1, dans laquelle ladite formulation comprend en outre un ou plusieurs ingrédients actifs choisis dans le groupe constitué par : les médicaments anti-inflammatoires stéroïdiens, les médicaments anti-inflammatoires non stéroïdiens, les médicaments antiallergiques, les bêta-bloquants, les inhibiteurs de la prostaglandine, les inhibiteurs de l'anhydrase carbonique, les agonistes alpha-adrénergiques, les médicaments antiviraux, les antibiotiques choisis dans le groupe constitué par : la lévofloxacine, l'ofloxacine, la tobramycine, l'azithromycine et leurs sels, les anesthésiques et les mydriatiques.

3. Formulation topique ophtalmique à utiliser selon la revendication 1, dans laquelle ladite formulation est administrée audit patient avec une dose de 0,100 à 0,540 mg/jour de nétilmicine.

4. Formulation topique ophtalmique à utiliser selon l'une quelconque des revendications 1 à 3, comprenant 0,1 à 0,5 % (poids/volume) de nétilmicine.

5. Formulation topique ophtalmique à utiliser selon l'une quelconque des revendications 2 à 4, dans laquelle lesdits un ou plusieurs médicaments anti-inflammatoires sont choisis dans le groupe constitué par : la dexaméthasone, le bromfénac, le népafénac et leurs sels.

6. Formulation topique ophtalmique à utiliser selon la revendication 5, dans laquelle un ou plusieurs médicaments anti-inflammatoires stéroïdiens sont la dexaméthasone et ses sels.

7. Formulation topique ophtalmique à utiliser selon la revendication 6, dans laquelle ladite formulation est administrée audit patient avec une dose de 0,03 à 0,18 mg/jour de dexaméthasone.

8. Formulation topique ophtalmique à utiliser selon la revendication 6 ou 7, comprenant 0,02 à 0,4 % (poids/volume) de dexaméthasone.

9. Formulation topique ophtalmique à utiliser selon la revendication 1, dans laquelle ladite pathologie oculaire est choisie dans le groupe constitué par : une infection oculaire, un traumatisme ou abrasion oculaire postopératoire et sécheresse oculaire.

10. Formulation topique ophtalmique à utiliser selon la revendication 9, dans laquelle ladite infection oculaire est choisie dans le groupe constitué par : la blépharite, la conjonctivite bactérienne et la kératite.

11. Formulation topique ophtalmique à utiliser selon l'une quelconque des revendications 1 à 10, dans laquelle ladite formulation comprend de la nétilmicine et de la dexaméthasone ou leurs sels.

12. Formulation topique ophtalmique comprenant de la gomme xanthane, de la nétilmicine et de la dexaméthasone pour une utilisation dans le traitement d'une pathologie oculaire ayant un état inflammatoire du segment avant de l'œil en présence d'une infection bactérienne, dans laquelle ladite formulation comprend de la gomme xanthane en une quantité allant de 0,7 à 5,0 % (poids/volume) et dans laquelle ladite formulation est administrée à un patient qui en a besoin avec une dose de 0,100 à 0,540 mg/jour de nétilmicine et de 0,03 à 0,18 mg/jour de dexaméthasone.

13. Formulation topique ophtalmique à utiliser selon la revendication 12, comprenant 0,1 à 0,5 % (poids/volume) de nétilmicine.

14. Formulation topique ophtalmique à utiliser selon la revendication 12 ou 13, comprenant 0,02 à 0,4 % (poids/volume) de dexaméthasone.

15. Formulation topique ophtalmique à utiliser selon la revendication 12, dans laquelle ladite pathologie oculaire est choisie dans le groupe constitué par : une infection oculaire, et un traumatisme ou abrasion oculaire postopératoire.

16. Formulation topique ophtalmique à utiliser selon la revendication 15, dans laquelle ladite infection oculaire est choisie dans le groupe constitué par : la blépharite, la conjonctivite bactérienne et la kératite.

17. Formulation topique ophtalmique à utiliser selon l'une quelconque des revendications 1 à 10, comprenant :
| Composant | Quantité % (poids/volume) |
|---|---|
| Nétilmicine et ses sels | 0,30 en tant que base |
| Dexaméthasone et ses sels | 0,10 en tant qu'alcool |
| Gomme xanthane | 0,7 - 5,0 |
| Monohydrate de phosphate d'hydrogène de sodium | 0,1465 |
| Phosphate disodique dodécahydraté | 0,5000 |
| Citrate de sodium | 2,1000 |
| Eau purifiée | q. s. pour 100 ml |
ou comprenant :
| Composant | Quantité % (poids/volume) |
|---|---|
| Nétilmicine et ses sels | 0,3 en tant que base |
| Gomme xanthane | 0,7 - 5,0 |
| Agents inducteurs de viscosité | q. s. pour 100 ml |
| Tampon | |
| Agents isotonisants | |
| Eau purifiée | |

18. Formulation topique ophtalmique à utiliser selon l'une quelconque des revendications 12 à 16, comprenant :
| Composant | Quantité % (poids/volume) |
|---|---|
| Nétilmicine et ses sels | 0,30 en tant que base |
| Dexaméthasone et ses sels | 0,10 en tant qu'alcool |
| Gomme xanthane | 0,7 - 5,0 |
| Monohydrate de phosphate d'hydrogène de sodium | 0,1465 |
| Phosphate disodique dodécahydraté | 0,5000 |
| Citrate de sodium | 2,1000 |
| Eau purifiée | q. s. pour 100 ml |
